# EUROPEAN PATENT APPLICATION

(11) **EP 4 140 500 A1**
(43) Date of publication of application: **01.03.2023**
(21) Application number: 20932686.7
(22) Date of filing: 07.12.2020
(51) Int. Cl.: A61K 39/00, A61K 9/14, A61K 31/7088, A61K 47/54, A61K 47/20, A61P 31/04, A61P 31/12, A61P 35/00

(54) **APPLICATION OF HETEROCYCLIC COMPOUND CONTAINING AT LEAST TWO SULFUR ATOMS IN PREPARING NANO-VACCINE AND PREPARED NANO-VACCINE**

(30) Priority: 24.04.2020 CN 202010330996
(71) Applicant: Suzhou Weast Biotechnology Co., Ltd., Suzhou, Jiangsu 215123 (CN)
(72) Inventor: YANG, Huanghao, Fuzhou, Fujian 350108 (CN); LI, Juan, Fuzhou, Fujian 350108 (CN); ZHANG, Da, Fuzhou, Fujian 350108 (CN); LIU, Xiaolong, Fuzhou, Fujian 350108 (CN)
(74) Representative: De Clercq & Partners
(86) International application number: PCT/CN2020/134162
(87) International publication number: WO 2021/212858

(57) **Abstract**

The present invention pertains to the technical field of immunotherapy or disease prevention and treatment with vaccines, in particular to a heterocyclic compound containing two or more sulfur atoms and an application thereof in preparing a nano-vaccine. Provided is the application of the heterocyclic compound containing at least two sulfur atoms and capable of being covalently or non-covalently linked to a polypeptide in preparing the nano-vaccine. A nanoparticle prepared by self-assembly of the compound and an antigen can enter the dendritic cytoplasm in a membrane-crossing manner, thereby improving the uptake efficiency of the antigen and an immune adjuvant. In the process of entering a cell, the nano-vaccine can effectively avoid or reduce biodegradation of the antigen or nucleic acid adjuvant caused by enzymes in lysosomes, and therefore the nano-vaccine can efficiently activate the dendritic cells and improve the cross-presentation of the antigen, thereby effectively activating CD8+T cells and promoting T cell proliferation. Therefore, the nano-vaccine can prevent tumor cell proliferation and virus infection by efficient immune activation and immune regulation.

## Description

This application claims the benefit of priority to Chinese patent application No. 202010330996.8, filed on 24 April 2020 and entitled "APPLICATION OF HETEROCYCLIC COMPOUND CONTAINING AT LEAST TWO SULFUR ATOMS IN PREPARING NANO-VACCINE AND PREPARED NANO-VACCINE", the entire disclosure of which is incorporated herein by reference.

### TECHNICAL FIELD

The present disclosure relates to the technical field of immunotherapy or vaccine prevention and/or treatment, in particular to an application of a heterocyclic compound containing two or more sulfur atoms in preparing nano-vaccine and the prepared nano-vaccine.

### BACKGROUND

In recent years, the researches and development of immune vaccines has made significant progress in the comprehensive prevention and/or treatment of cancer and virus infections, and has relatively high clinical application value in respect of anti-tumor or anti-virus infection. At present, various countries have successively developed various anti-tumor or anti-virus vaccines for disease prevention or treatment and have entered clinical phase I, II. Some have been approved for clinical use in the United States and Europe. However, it has been challenging to activate antigen-presenting cells and enhance cross-presentation. For example, the ingestion efficiency of dendritic cells by simply mixed adjuvants and antigen preparations is not high, and the ingested antigen or adjuvant, after entering cell organelles such as intracellular lysosomes, is more likely to be degraded by enzymes to reduce or lose the activation of dendritic cells, thereby weakening the effect of antigen cross-presentation, being unable to effectively activate the body's immune system to deal with external or internal tumors or virus infections, and even being unable to cope with certain types of tumors or virus infections.

Efficient antigen presentation and its activation of the immune system to play an immunomodulatory role mainly depend on whether the antigen-presenting cells can efficiently take in the antigen and whether they can be effectively activated to exert their cross-presentation effect on the antigen. The existing nano-vaccine enters the dendritic cells mainly in an endocytosis-lysosome manner, that is, after being endocytosed by the dendritic cells, it will form endosomes or vesicles, and then fuse with intracellular lysosomes. The nuclease or proteolytic enzyme in the lysosome will cleave or hydrolyze the nucleic acid adjuvant or antigen peptide released by the nano-vaccine, reduce the stimulation and pro-activation effect of the nucleic acid adjuvant on dendritic cells, affect its effect on the cross-presentation of antigen peptide and on the activation and proliferation of immature T lymphocytes, weaken the body's cellular immune response to antigen peptides, and result in poor tumor immunotherapy.

In attempts to address these limitations, liposome is used as a carrier of mRNA and peptide vaccines to protect the active ingredient from degradation. However, liposome is internalized via endosomal escape, which is an endocytic pathway, the endo- and lysosomal trapping and degradation are inevitable. In addition, the polyethylene glycol on the surface of liposomes can effectively resist or hinder the endocytosis of macrophages and other lymphocytes, the efficiency of antigen uptake and the activation ratio of lymphocytes are further reduced, thereby reducing the cross-presentation of antigens. Therefore, there is an urgent need to develop a new antigen uptake and presentation mode to efficiently activate lymphocytes and improve cross-presentation efficiency.

### SUMMARY

In view of this, the technical problem to be solved by the present disclosure is to provide an application of a heterocyclic compound containing disulfide bonds in the preparation of nano-vaccine and the prepared nano-vaccine. The nano-vaccine comprises thiol compound, tumor neogenetic polypeptide antigen or virus polypeptide antigen and nucleic acid adjuvant, and is self-assembled by electrostatic force, hydrogen bond and van der Waals force. The vaccine enters the cytoplasm of dendritic cells in a membrane-crossing pathway with high efficiency, thus reducing the degradation of polypeptide antigen and nucleic acid by protease or nuclease in lysosome.

The present disclosure provides an application of a compound of formula I in the preparation of nano-vaccine;

A-L-F Formula I

wherein A is a heterocyclic group comprising two or more sulfur (S) atoms;
L is none or a linking group;
F is a group capable of being covalently or non-covalently linked to a polypeptide.

The compound of formula I comprises at least two sulfur atoms, which are able to self-assemble with nucleic acid adjuvants and neoantigen peptides to form a nano-vaccine. The F group is connected to the polypeptide, and the A group is used for disulfide exchange reactions on the cell membrane and mediates the nano-vaccine to cross the membrane and directly enter the cytoplasm of dendritic cell. This pathway of entering the cell does not go in the endocytosis-lysosome manner. After entering the cell, the nano-vaccine is depolymerized by cytoplasmic buffer to release nucleic acid adjuvant and neoantigen peptide therein. The released nucleic acid adjuvant can more effectively stimulate the maturation of dendritic cells, enhance the cross-presentation of dendritic cells to neoantigen peptides, enhance the ability to activate and promote proliferation of immature T lymphocytes, enhance the body's specific cellular immune response to tumor cells expressing the neoantigen peptides, and enhance the specific cellular immunotherapy of T cell-dependent tumor.

In the present disclosure, the heterocyclic group in A is a 4- to 8-membered ring.

In some embodiments, the A is: 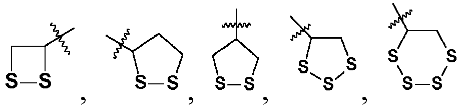 or

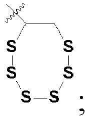

In the present disclosure, L is -(CH₂)ₙ-, 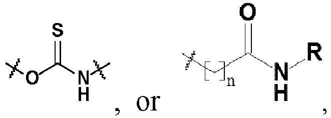 wherein n=1~5; R is -H, C₁₋₅ alkyl group, 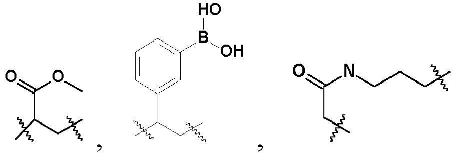 or 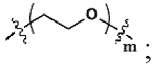 m=1~50.

In some embodiments, the L is:

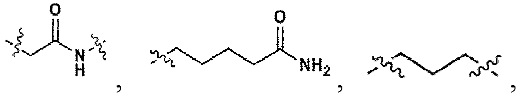

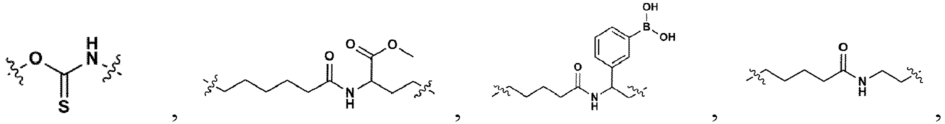

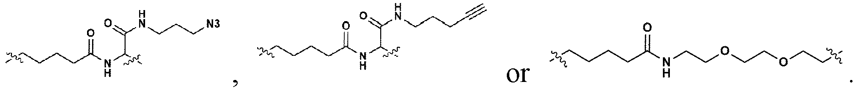

In the present disclosure, the covalent connection may be selected from: click chemistry reaction and cross-linking reaction; the non-covalent connection includes: electrostatic interaction, hydrophobicity, and affinity.

The F groups that may be connected to the polypeptide through click chemistry reactions include:

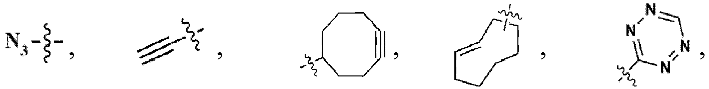

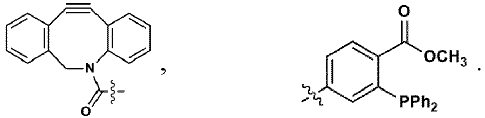

The F groups that may be connected to the polypeptide through a cross-linking reaction include:

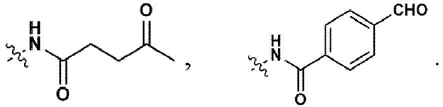

The F groups that may form a covalent connection with the polypeptide through other reaction methods include:

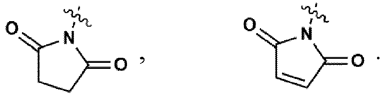

The F groups that may be connected to the polypeptide through electrostatic interaction include:

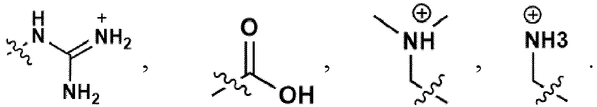

The F groups that may be linked to the polypeptides through hydrophobic interactions include:

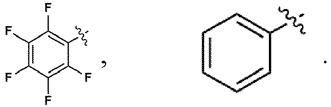

The F groups that may be linked to polypeptides through affinity include:

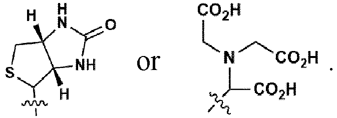

In some embodiments, the F is:

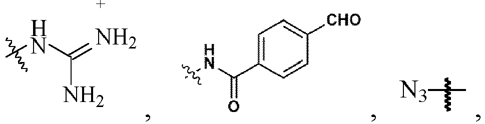

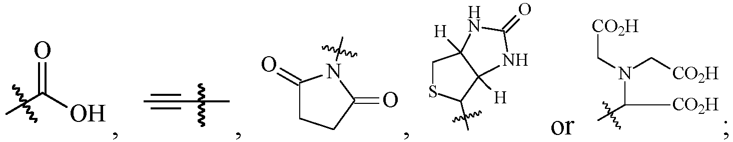

In some embodiments, the A is 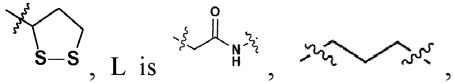 or 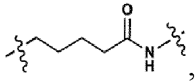 F is

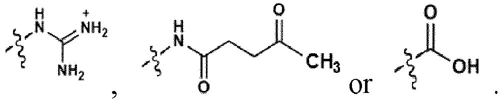

In some specific embodiments, the compound of formula I is

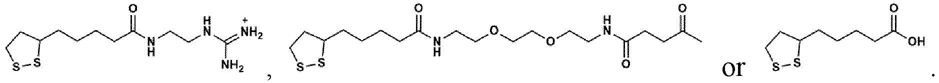

The formula I compounds provided by the present disclosure was used to prepare nanoparticles with polypeptides. Notably, to get the appropriate-size nano-vaccine for efficient cellular uptake, formula I compounds should be selected carefully according to polypeptides.

The present disclosure also provides a nanoparticle, the raw material of which includes a compound of formula I, a nucleic acid adjuvant and an antigen peptide;

A-L-F Formula I

wherein, A is a heterocyclic group comprising two or more S atoms;
L is none or a linking group;
F is a group capable of being covalently or non-covalently linked to a polypeptide.

In the present disclosure, the antigen peptide is selected from tumor antigens, bacterial antigens, or virus antigens.

In the present disclosure, the tumor antigen is a tumor neoantigen, selected from neoantigens of bladder cancer, blood cancer, bone cancer, brain cancer, breast cancer, central nervous system cancer, cervical cancer, colon cancer, endometrial cancer, esophageal cancer, gallbladder cancer, gastrointestinal cancer, external genital cancer, urogenital cancer, head cancer, kidney cancer, laryngeal cancer, liver cancer, lung cancer, muscle tissue cancer, neck cancer, oral or nasal mucosal cancer, ovarian cancer, pancreatic cancer, prostate cancer, skin cancer, spleen cancer, small intestine cancer, large intestine cancer, stomach cancer, testicular cancer and/or thyroid cancer;

In the present disclosure, the virus antigen is influenza virus antigen, Ebola virus antigen, coronavirus antigen, hepatitis virus antigen, mumps virus antigen, varicella-zoster virus antigen, measles virus antigen, rubella virus antigen, influenza virus antigen and/or avian influenza virus antigen;

The coronavirus is COVID-19, SARS; the hepatitis virus is hepatitis A virus, hepatitis B virus or hepatitis C virus; the influenza virus is influenza A virus, influenza B virus or influenza C virus, wherein types of hemagglutinin protrusions of the influenza A virus include H1, H2, H3, H4, H5, H6, H7, H8, H9, H10, H11, H12, H13, H14 or H15, types of nerve ammonia acid enzyme protrusions are N1, N2, N3, N4, N5, N6, N7, N8 or N9.

In the present disclosure, the bacterial antigens are binding mycobacterial antigens, staphylococcal antigens, streptococcal antigens, pneumococcal antigens, bacillus anthracis antigens, diphtheria bacillus antigens, proteus antigens, pertussis bacillus antigen, vibrio cholerae antigens, meningococcal antigens and/or typhoid bacilli antigens.

In some specific embodiments, the tumor antigen is a liver cancer antigen, specifically a liver cancer H22 polypeptide antigen, and more specifically, having an antigen sequence of HTDAHAQAFAALFDSMH. The virus antigen is a coronavirus antigen, specifically a novel coronavirus antigen; more specifically, having an antigen sequence of SYYSLLMPI, RYVLMDGSI, AYANSVFNI, TYASALWEI or GYLKLTDNV

In an embodiment of the present disclosure, the isoelectric point of the antigen is less than 6.5.

In an embodiment of the present disclosure, the nucleic acid adjuvant is CpG-ODN, PolyI:C.

In the present disclosure, a Cy3 fluorochrome is attached to the N-terminal of the polypeptide antigen, and a FAM fluorochrome is conjugated to the 3'-terminal of the nucleic acid adjuvant. The fluorochrome is used to define subcellular localization of the nano-vaccine in dendritic cells. Our data shows that fluorochrome has no impact on the activation of dendritic cells.

In the embodiment of the present disclosure, in the nanoparticle, a molar ratio of the compound of formula I, the nucleic acid adjuvant and the antigen peptide is (50-100): (0.5-1): (20-50). In some specific embodiments, a molar ratio of the compound of formula I, the nucleic acid adjuvant and the antigen peptide in the nanoparticle is 50:0.5:20.

The guanidine group in the positively charged compound of formula I and the phosphate group in the negatively charged nucleic acid adjuvant form a salt bridge, and then self-assembly prepare and synthesize the nanoparticle of the present disclosure with the amino acid of the negatively charged neoantigen peptide via the electrostatic interaction between molecules. The nucleic acid adjuvant may be CpG-ODN (mouse or human), or PolyI:C (mouse or human); the isoelectric point (pI) of the neoantigen peptide is less than 6.

In some embodiments, a preparation method of a nanoparticle includes: a preparation method of a nano-vaccine comprising: mixing the compound of formula I, nucleic acid adjuvant and polypeptide antigen with a TM buffer (pH 7.4), stirring at 37° C, 100 rpm for 15 minutes, and then dialyzing in deionized water, to prepare a solution containing nanoparticles.

A volume ratio of the total volume of the compound of formula I, the nucleic acid adjuvant and the polypeptide antigen solution to the TM buffer is 1:60-70.

In a specific embodiment, the compound of formula I in methanol solution (146.2 mM), nucleic acid adjuvant solution (100 mM), and polypeptide antigen solution (2.5 mg/mL) are mixed in a volume ratio of 1:1:1, and then the mixture is mixed with a TM buffer (pH 7.4) in a volume ratio of 1:60-70.

The present disclosure also provides a nano-vaccine, comprising the nanoparticle of the present disclosure and an adjuvant acceptable in the nano-vaccine.

The acceptable adjuvants in the vaccine include solvents and osmotic pressure regulators. Preferably, the nano-vaccine prepared by the present disclosure includes the nanoparticle of the present disclosure and sodium chloride injection.

In the present disclosure, the dendritic cells internalize the nano-vaccine via thiol-mediated cellular uptake, then the nano-vaccine is disassembled by cytoplasmic buffer to release polypeptide antigens, nucleic acid adjuvants, thereby efficiently activating dendritic cells, improving their antigen cross-presentation, and effectively activating CD8+ T cells.

The present disclosure also provides a method for prevention and/or treatment of tumor, comprising: administering the nano-vaccine of the present disclosure.

The present disclosure also provides a method for prevention and/or treatment of bacterial infection and/or virus infection, comprising: administering the nano-vaccine of the present disclosure.

The present disclosure pertains to the technical field of immunotherapy or disease prevention and/or treatment with vaccines, in particular to a heterocyclic compound comprising two or more sulfur atoms and an application thereof in preparing a nano-vaccine. The present disclosure provides an application of the heterocyclic compound comprising at least two sulfur atoms and capable of being covalently or non-covalently linked to a polypeptide in preparing a nano-vaccine. A nanoparticle prepared by self-assembly of the compound, a nucleic acid adjuvant and an antigen can enter the dendritic cytoplasm in nonendocytic pathway, thereby improving the uptake efficiency of the antigen and an immune adjuvant. In the process of entering a cell, the nano-vaccine can effectively avoid or reduce biodegradation of the antigen or nucleic acid adjuvant caused by enzymes in lysosomes, and therefore the nano-vaccine can efficiently activate the dendritic cells and improve the cross-presentation of the antigen, thereby effectively activating CD8+T cells and promoting T cell proliferation. Therefore, the nano-vaccine of the present disclosure can prevent tumor cell proliferation and virus infection by efficient immune activation and immune regulation.

### BRIEF DESCRIPTION OF THE DRAWINGS

FIG. 1 shows a schematic illustration of nano-vaccine preparation and application of anti-tumor;
FIG. 2 shows the scanning electron microscopy images of the nano-vaccine of Example 3;
FIG. 3 shows the size distribution of the nano-vaccine of Example 3 and the control nano-vaccine;
FIG. 4 shows confocal fluorescence microscopy images of the nano-vaccine subcellular distribution in dendritic cells. Nano-vaccine of Example 3 was incubated with dendritic cells for 30 minutes, wherein the polypeptide antigens were labeled with red fluorescence, the nucleic acid adjuvants were labeled with green fluorescence, and the scale is 20 µm;
FIG. 5 shows confocal fluorescence microscopy images of the nano-vaccine subcellular distribution of Example 3 in dendritic cells, wherein the lysosomes were labeled with green fluorescence, the polypeptide antigens were labeled with red fluorescence, and the scale is 20 µm;
FIG. 6 shows confocal fluorescence microscopy images of the nano-vaccine subcellular distribution in dendritic cells. Nano-vaccine of Example 4 was incubated with dendritic cells for 30 minutes, wherein the polypeptide antigens were labeled with red fluorescence, the nucleic acid adjuvants were labeled with green fluorescence, and the scale is 10 µm;
FIG. 7 shows confocal fluorescence microscopy images of the nano-vaccine subcellular distribution of Example 4 in dendritic cells, wherein the lysosomes were labeled with green fluorescence, the polypeptide antigens were labeled with red fluorescence, and the scale is 10 µm;
FIG. 8 shows confocal fluorescence microscopy images of the nano-vaccine subcellular distribution in dendritic cells. Nano-vaccine of Example 5 was incubated with dendritic cells for 30 minutes, wherein the polypeptide antigens were labeled with red fluorescence, the nucleic acid adjuvants were labeled with green fluorescence, and the scale is 10 µm;
FIG. 9 shows confocal fluorescence microscopy images of the nano-vaccine subcellular distribution of Example 5 in dendritic cells, wherein the lysosomes were labeled with green fluorescence, the polypeptide antigens were labeled with red fluorescence, and the scale is 10 µm;
FIG. 10 shows the activation of dendritic cells after being treated with the nano-vaccine of Example 3 for 3 days;
FIG. 11 shows the proliferation of dendritic cells activated by the nano-vaccine prepared in Example 3 after co-cultivation with CD8+T cells for 2 days;
FIG. 12 shows a mouse tumor growth curve of H22 murine liver cancer treated by the nano-vaccine of Example 3.

### DETAILED DESCRIPTION

The present disclosure provides an application of a heterocyclic compound comprising at least two S atoms in the preparation of nano-vaccine and the prepared nano-vaccine. Those skilled in the art may learn from the content of this disclosure and appropriately improve process parameters to realize. In particular, it should be pointed out that all similar replacements and modifications are obvious to those skilled in the art, and they are all deemed to be included in the present disclosure. The method and application of the present disclosure have been described through the preferred embodiments. It is obvious that relevant personnel can modify or appropriately change and combine the methods and applications herein without departing from the content, spirit and scope of the present disclosure to realize and apply the technology of present disclosure.

The materials used in the present disclosure are all common commercially available products, all of which can be purchased in the market.
(1) Chemical reagents: all nucleic acid adjuvants (see Table 1 for sequences) were prepared by Sangon Biotech (Shanghai) Co., Ltd. and synthesized by HPLC purification method. LysoTracker and Hoechst 33342 were purchased from Thermo Fisher Scientific Co., Ltd. of the United States of America. Ultrapure water was prepared by Milli-Q water purification system (18.2M Ω ).
(2) Cell lines and cell culture: murine hepatocarcinoma cells H22 were purchased from the American Type Culture Collection (ATCC). H22 cells were cultured in DMEM medium (HyClone) supplemented with a final concentration of 10% fetal bovine serum (Gibco) and a final concentration of 100 IU/mL penicillin-streptomycin, in 5% CO₂ at 37° C.
(3) The required murine nucleic acid adjuvant CpG-ODN was used to activate dendritic cells. The base sequence is as follows:

**Table 1 DNA sequences of CpG-ODN used in the examples**

| name | Base sequence (5' →3') |
|---|---|
| FAM fluorescence labeled CpG-ODN | TCCATGACGTTCCTGACGTT-FAM |
| Unlabeled CpG-ODN | TCCATGACGTTCCTGACGTT |

### Compound:

Compound 1: 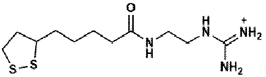 synthesized in Example 1;
Compound 2: 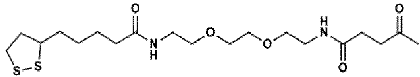 synthesized in Example 2;
Compound 3: 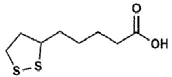 purchased from Macleans Biochemical Technology Co., Ltd.

The present disclosure was further illustrated by following examples.

### Example 1

The dichloromethane solution comprising N,N'-carbonyldiimidazole and lipoic acid was gradually dropped into the dichloromethane comprising ethylenediamine kept at 0°C. The reaction mixture was stirred 1 h at 0 °C and 1 h at room temperature. The organic layer was dried over anhydrous Na₂SO₄ and concentrated under reduced pressure, yielding substance as oil. The oily substance was dissolved in dichloromethane and 1H-pyrazole-1-carboxamidine hydrochloride was added. The suspension was stirred at room temperature until complete consumption of starting reagent. Solvent was removed under reduced pressure and the residue was dissolved in methanol. Ether was added to induce precipitation, and then the precipitate was washed with ether, yielding the final product Compound 1.

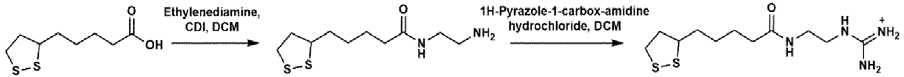

### Example 2

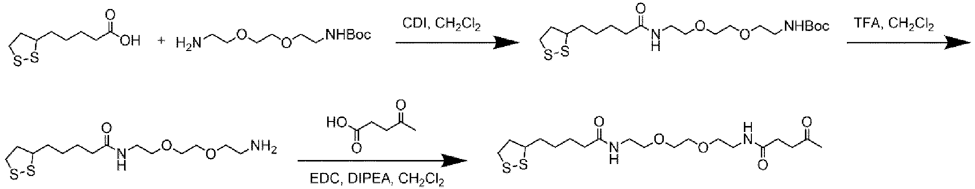

The anhydrous CH₂Cl₂ solution of molecule 1 was activated by adding 1,1-carbonyldiimidazole (CDI), and then molecule 2 was added for condensation reaction to obtain molecule 3. Trifluoroacetic acid (TFA) was added to the CH₂Cl₂ solution of molecule 3 to remove the tert-butoxycarbonyl protecting group to obtain molecule 4. Molecule 4, levulinic acid, 1-ethyl-(3-dimethylaminopropyl) carbodiimide hydrochloride (EDC), and N, N-diiso propylethylamine (DIPEA) were added in anhydrous CH₂Cl₂, molecule 5 was obtained through condensation reaction.

### Example 3

Nano-vaccine preparation method: compound 1 (concentration: 146.2mM) prepared in Example 1, 100 mM nucleic acid adjuvant (CpG-ODN), and 2.5 mg/mL polypeptide antigen (mouse liver cancer cell H22 polypeptide antigen, amino acid sequence: HTDAHAQAFAALFDSMH, N terminal connected to a Cy3 label, and isoelectric point: 5.71) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain nano-vaccine.

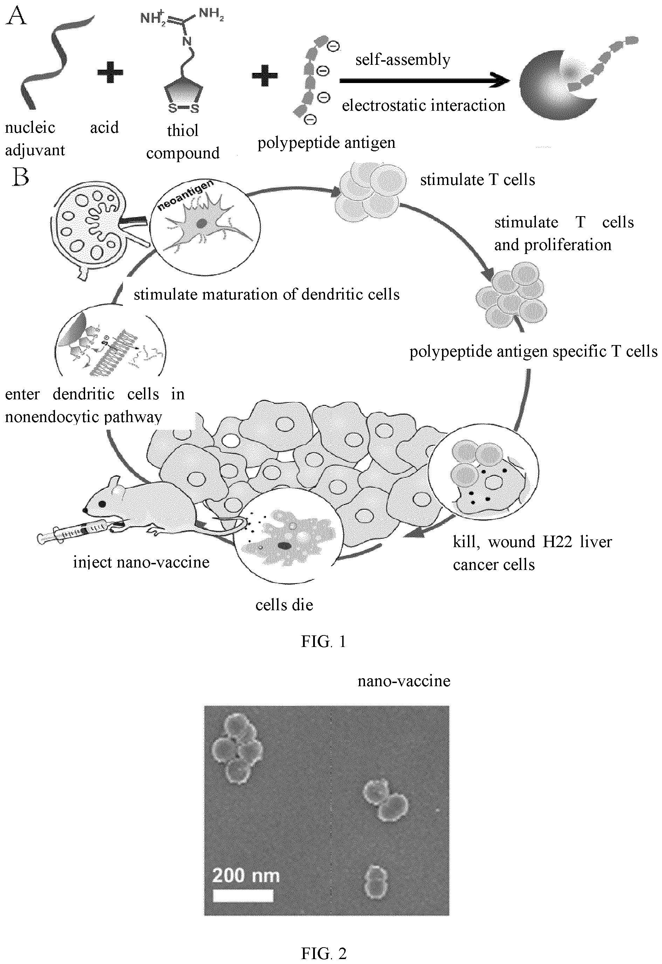

The dynamic light scattering analysis and the scanning electron microscope images indicated the successful preparation of nano-vaccine. The results show that CpG-ODN, polypeptide antigen and compound 1 formed spherical nano-vaccines in the TM buffer (pH=7.5), with a particle size of about 100 nm (as shown in FIG. 2a). The concentration of the active ingredient CpG-ODN was 8.8 µg/mL, the concentration of polypeptide antigen was 6.11 µg/mL.

### Comparative example

The compound 1 (concentration: 146.2 mM) prepared in Example 1, 100 mM nucleic acid adjuvant (CpG-ODN), 2.5 mg/mL control polypeptide antigen (amino acid sequence: YKYRYLRHGKLR, N-terminal connected to a Cy3 label, and the isoelectric point: 10.55) were mixed at a volume ratio of 1:1:1, Next, a 60 to 70 times volume of TM buffer solution (pH 7.4) to the mixture was added, then stirred at 37 °C for 15 min and dialyzed in deionized water for 24 h to remove unloaded nucleic acid adjuvant or polypeptide antigen, to finally obtain comparative nano-vaccine. The dynamic light scattering analysis showed that the hydrodynamic size of the nano-vaccine was about 100 nm, while the hydrodynamic size of the comparative nano-vaccine was about 600 nm. This result indicated that due to the difference in the isoelectric point of the polypeptide, the size of the formed comparative nano-vaccine was too large to achieve a high-efficiency cell uptake.

### Example 4

Nano-vaccine preparation method: compound 2 prepared in Example 2 (concentration: 146.2 mM), 100 mM nucleic acid adjuvant (CpG-ODN), and 2.5 mg polypeptide antigen (new coronavirus antigen polypeptide 1, amino acid sequence: FYYVWKSYV, N terminal connected to a Cy3 label, isoelectric point: 8.43) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain the nano-vaccine.

The dynamic light scattering analysis and the scanning electron microscope images indicated the successful preparation of nano-vaccine. The concentration of active ingredient CpG-ODN was 8.8 µg/mL, and the concentration of polypeptide antigen was 6.11 µg/mL.

### Example 5

Nano-vaccine preparation method: compound 3 (concentration: 146.2 mM), 100 mM nucleic acid adjuvant (CpG-ODN), and 2.5mg polypeptide antigen (new coronavirus polypeptide antigen 2, amino acid sequence: KYTQLCQYL, N-terminal connected to a Cy3 label, isoelectric point: 8.5) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain the nano-vaccine.

The dynamic light scattering analysis and the scanning electron microscope images indicated the successful preparation of nano-vaccine. The concentration of active ingredient CpG-ODN was 8.8 µg/mL, and the concentration of polypeptide antigen was 6.11 µg/mL.

### Example 6

Nano-vaccine preparation method: compound 1 (concentration: 146.2 mM) prepared in Example 1, 100 mM nucleic acid adjuvant (CpG-ODN), and 2.5 mg polypeptide antigen (new coronavirus polypeptide antigen 3, amino acid sequence: SYYSLLMPI, isoelectric point: 5.55) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain the nano-vaccine.

The dynamic light scattering analysis and the scanning electron microscope images indicated the successful preparation of nano-vaccine. The concentration of active ingredient CpG-ODN was 7.8 µg/mL, and the concentration of polypeptide antigen was 7.41 µg/mL.

### Example 7

Nano-vaccine preparation method: compound 1 (concentration: 146.2 mM) prepared in Example 1, 100 mM nucleic acid adjuvant (CpG-ODN), and 2.5 mg polypeptide antigen (new coronavirus polypeptide antigen 4, amino acid sequence: RYVLMDGSI, isoelectric point: 6.21) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain the nano-vaccine.

The dynamic light scattering analysis and the scanning electron microscope images indicated the successful preparation of nano-vaccine. The concentration of active ingredient CpG-ODN was 7.1 µg/mL, and the concentration of polypeptide antigen was 5.82 µg/mL.

### Example 8

Nano-vaccine preparation method: compound 1 (concentration: 146.2 mM) prepared in Example 1, 100mM nucleic acid adjuvant (CpG-ODN), and 2.5 mg polypeptide antigen (new coronavirus polypeptide antigen 5, amino acid sequence: AYANSVFNI, isoelectric point: 5.55) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain the nano-vaccine.

The dynamic light scattering analysis and the scanning electron microscope images indicated the successful preparation of nano-vaccine. The concentration of active ingredient CpG-ODN was 9.1 µg/mL, and the concentration of polypeptide antigen was 7.21 µg/mL.

### Example 9

Nano-vaccine preparation method: compound 1 (concentration: 146.2 mM) prepared in Example 1, 100 mM nucleic acid adjuvant (CpG-ODN), and 2.5 mg polypeptide antigen (new coronavirus polypeptide antigen 6, amino acid sequence: TYASALWEI, isoelectric point: 3.75) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain the nano-vaccine.

The dynamic light scattering analysis and the scanning electron microscope images indicated the successful preparation of nano-vaccine. The concentration of active ingredient CpG-ODN was 7.1 µg/mL, and the concentration of polypeptide antigen was 7.67 µg/mL.

### Example 10

Nano-vaccine preparation method: compound 1 (concentration: 146.2 mM) prepared in Example 1, 100 mM nucleic acid adjuvant (CpG-ODN), and 2.5 mg polypeptide antigen (new coronavirus polypeptide antigen 7, amino acid sequence: GYLKLTDNV, isoelectric point: 6.21) were mixed at a volume ratio of 1:1:1. Next, a 60-70 times volume of TM buffer solution (pH 7.4) to the mixture was added, then stirred at 37 °C for 15 min and dialyzed in deionized water for 24 h to remove the unloaded nucleic acid adjuvant or polypeptide antigen, to finally obtain the nano-vaccine.

The dynamic light scattering analysis and the scanning electron microscope images indicated the successful preparation of nano-vaccine. The concentration of active ingredient CpG-ODN was 9.12 µg/mL, and the concentration of polypeptide antigen was 7.14 µg/mL.

### Effectiveness verification

### A. Laser confocal fluorescence imaging analysis of the internalization of nano-vaccine in dendritic cells:

Nano-vaccines prepared in Examples 3-10 with CpG-ODN and polypeptide antigens respectively labeled with FAM and Cy3 fluorescent dyes were co-cultured with immature dendritic cells in a glass-bottom dishes for 30 minutes, and then stained with Hoechst 33342. The subcellular location of nano-vaccines was observed by laser confocal fluorescence microscope.

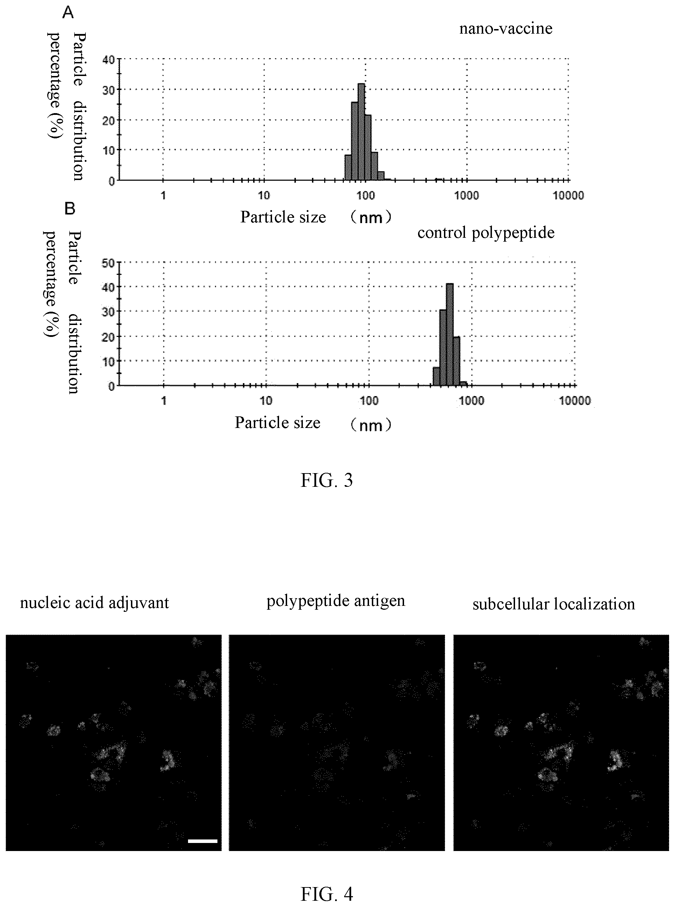

The nano-vaccine was co-cultured with dendritic cells for 30 minutes, and it was found that the nano-vaccine could quickly enter the dendritic cells. The cellular uptake of the nano-vaccine of Example 3 is shown in FIG. 3. In order to further verify that the vaccine enters the cell through a thiol-mediated uptake instead of an endocytic pathway, cells were stained with a lysosome dye (LysoTracker green). The nano-vaccine labeled with red fluorescence was mostly separated from the lysosomes and localized in the cytoplasm. This result suggested that the internalization pathway of nano-vaccine is the nonendocytic thiol-mediated uptake, and it was predicted that the nano-vaccine can significantly reduce the risk of degradation of CpG-ODN and polypeptide antigens in lysosomes (FIG. 4).

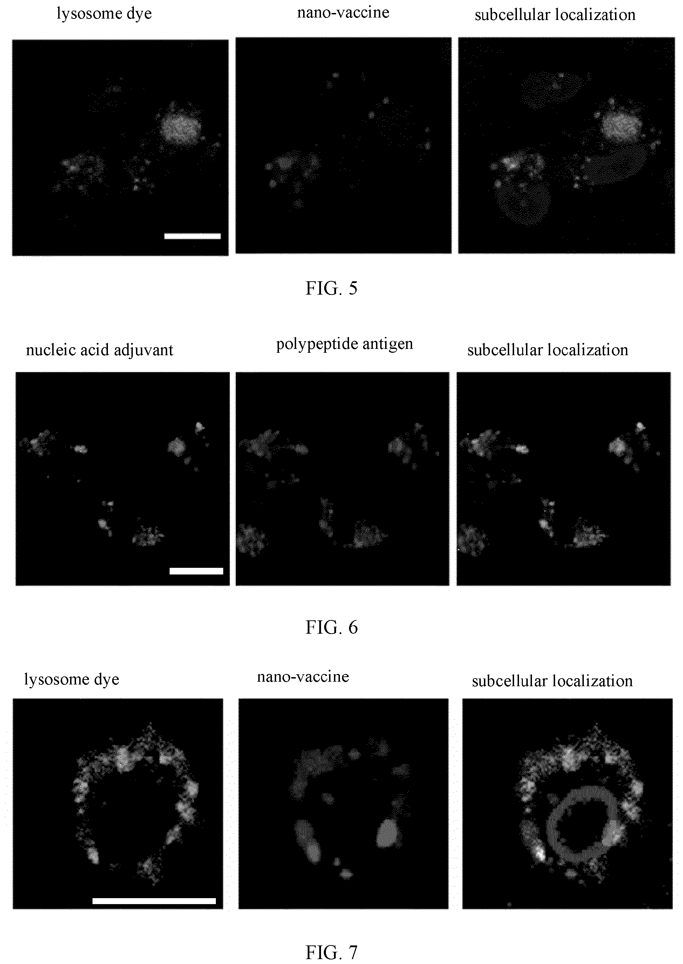

In addition, the cellular uptake of the nano-vaccine of Example 4 is shown in FIGS. 5-6; and the cellular uptake of the nano-vaccine of Example 5 is shown in FIGS. 7-8.

### B. Nano-vaccines induce the dendritic cells maturation efficiently:

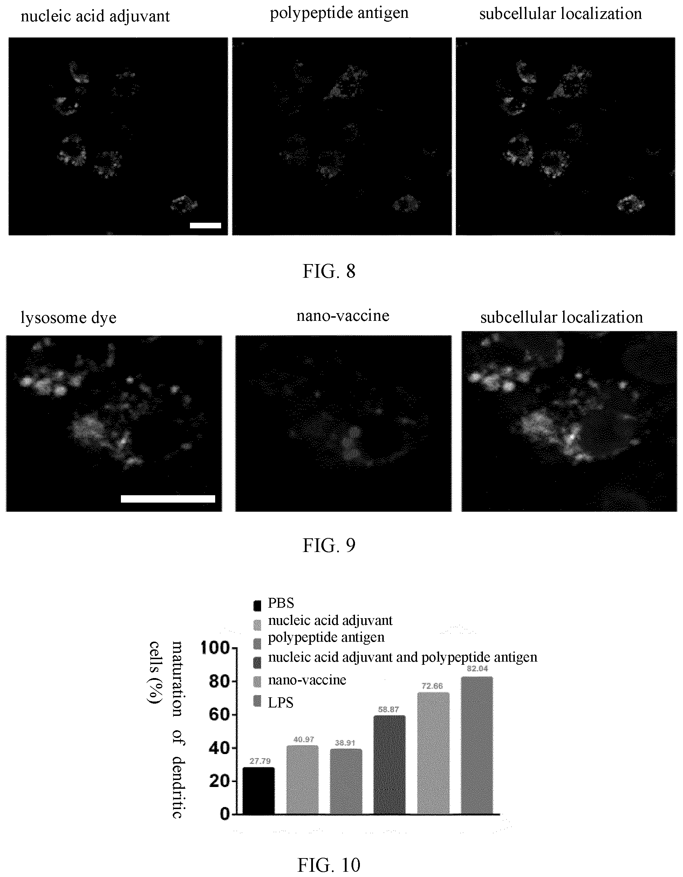

Dendritic cells were seeded in a 6-well plate and cultured at 37 °C. The dendritic cells were co-cultured with PBS buffer, CpG-ODN alone, polypeptide antigen, CpG-ODN and polypeptide antigen mixture, and nano-vaccine (Example 3) for 3 days, with LPS as a positive control. Then the dendritic cells were stained with antibodies of CD11c, CD80, CD86 labeled with fluorochrome, and flow cytometry was used for phenotype analysis. The data showed that the nano-vaccine treatment group had the highest proportion of mature dendritic cells compared with the other groups (PBS, CpG-ODN alone, polypeptide antigen, CpG-ODN and polypeptide antigen mixture), LPS was the positive control group (as shown in FIG. 9).

### C. Dendritic cells activated by nano-vaccine can effectively activate T cell maturation and proliferation:

To demonstrate that the nano-vaccine (Example 3) can efficiently activate the T cell-dependent immune response and enhance T cell maturation and cell proliferation, the dendritic cells of the different treatment groups in the above experiment B were co-cultured with immature mouse primary T cells inoculated into a 6-well plate for 2 days. Next, the T cells stimulated by dendritic cells were stained with fluorochrome-labeled CD8 antibody, and the phenotype was analyzed by flow cytometry. In addition, to evaluate the proliferation of CD8+ T cells, the T cells stimulated by dendritic cells were labeled with CFSE dye and co-cultured for 2 days. The fluorescence quantitation was analyzed using flow cytometry. The increased fluorescence intensity on the left side (P2) (as shown in FIG. 10) showed that dendritic cells treated with nano-vaccine can efficiently stimulate T cell maturation and effectively promote T cell proliferation.

### D. Mice immunized with nano-vaccine can effectively prevent tumors

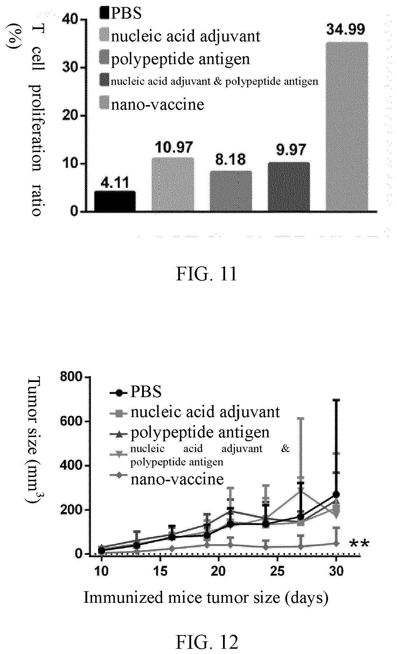

To evaluate the tumor prevention effects of the nano-vaccine synthesized in Example 3, BCLB/c male mice (5 weeks old) were inoculated with PBS buffer, CpG-ODN alone, polypeptide antigen, CpG-ODN and polypeptide antigen mixture, and nano-vaccine for once every 5 days. After five times immunizations, mice were transplanted with H22 tumors. The tumor size was measured every 3 days and the tumor growth curve was plotted. As shown in FIG. 11, the tumor growth of the mice treated with the nano-vaccine was obviously inhibited, and its inhibition efficiency was higher than that of other treatment groups. It was proved that the nano-vaccine can inhibit the growth of H22 liver cancer tumor more efficiently, and provide research basis for tumor immune prevention and/or treatment.

Therefore, nano-vaccine has good anti-tumor activity and has good clinical transformation and application prospects in tumor immunotherapy.

The above are only the preferred embodiments of the present disclosure. It should be pointed out that for those of ordinary skill in the art, without departing from the principle of the present disclosure, various improvements and modifications can be made, and these improvements and modifications should also be considered the protection scope of the present disclosure.

## Claims

1. An application of a compound of formula I in preparation of nano-vaccine;
A-L-F Formula I
**characterized in that**, A is a heterocyclic group comprising two or more S atoms;
L is none or a linking group;
F is a group capable of being covalently or non-covalently linked to a polypeptide.

2. The application according to claim 1, **characterized in that** the heterocyclic group in A is a 4- to 8-membered ring.

3. The application according to claims 1 or 2, **characterized in that** the L is -(CH₂)ₙ-, 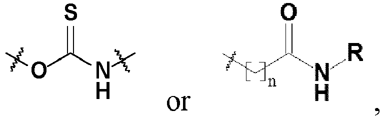 wherein n=1~5; R is -H, C₁₋₅ alkyl group, 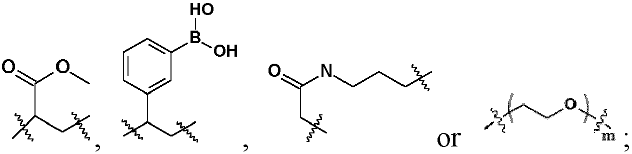 m=1~50.

4. The application according to any one of claims 1 to 3, **characterized in that** the F is:

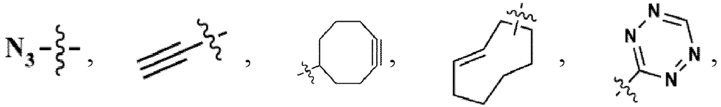

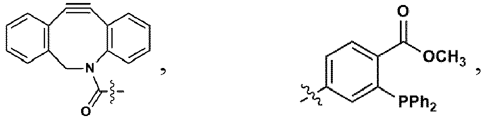

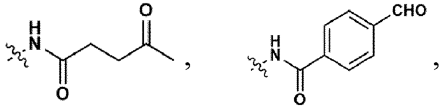

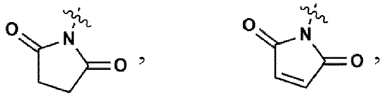

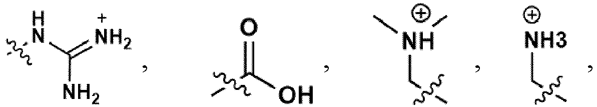

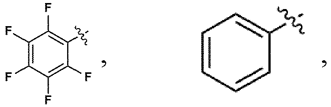

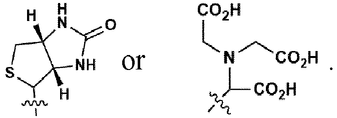

5. The application according to claim 1, **characterized in that** the A is: 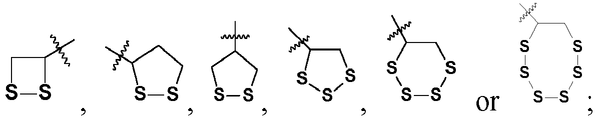 the F is: 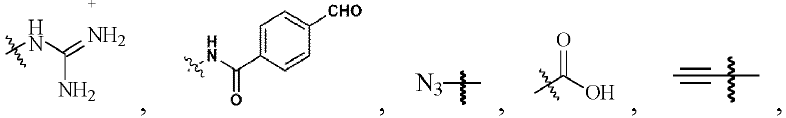 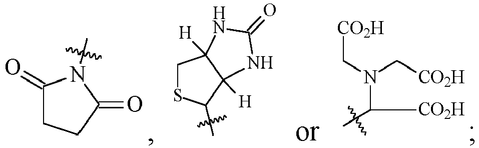 the L is:

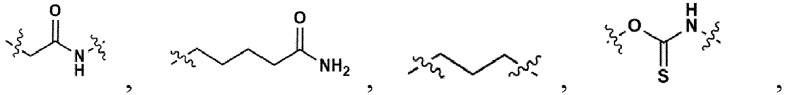

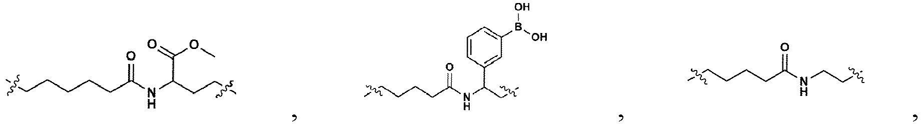

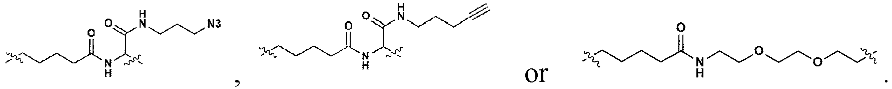

6. The application according to claim 1, **characterized in that** the compound of formula I is: 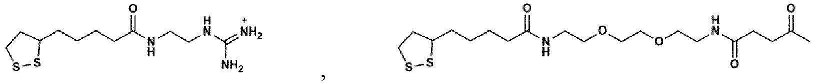 or

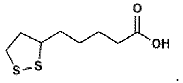

7. A nanoparticle, having a raw material comprising a compound of formula I and an antigen peptide;
A-L-F Formula I
**characterized in that**, A is a heterocyclic group comprising two or more S atoms;
L is none or a linking group;
F is a group capable of being covalently or non-covalently linked to a polypeptide.

8. The nanoparticle according to claim 7, **characterized in that** the antigen peptide is selected from tumor antigens, bacterial antigens or virus antigens.

9. The nanoparticle according to claim 8, **characterized in that** the tumor antigen is a tumor neoantigen selected from a neoantigen of bladder cancer, blood cancer, bone cancer, brain cancer, breast cancer, central nervous system cancer, cervical cancer, colon cancer, endometrial cancer, esophageal cancer, gallbladder cancer, gastrointestinal cancer, external genital cancer, urogenital cancer, head cancer, kidney cancer, laryngeal cancer, liver cancer, lung cancer, muscle tissue cancer, neck cancer, oral or nasal mucosa cancer , ovarian cancer, pancreatic cancer, prostate cancer, skin cancer, spleen cancer, small intestine cancer, large intestine cancer, gastric cancer, testicular cancer and/or thyroid cancer;
the virus antigen is influenza virus antigen, Ebola virus antigen, coronavirus antigen, hepatitis virus antigen, mumps virus antigen, varicella-zoster virus antigen, measles virus antigen, rubella virus antigen, influenza virus antigen and/or Avian influenza virus antigen;
the bacterial antigen is binding mycobacterial antigen, staphylococcal antigen, streptococcal antigen, pneumococcal antigen, anthracis antigen, diphtheria antigen, proteus antigen, pertussis antigen, vibrio cholerae antigen, meningococcal antigen and/or typhoid bacillus antigen.

10. The nanoparticle according to claim 7, **characterized in that** the nanoparticle further comprises a nucleic acid adjuvant, and the nucleic acid adjuvant is CpG-ODN, Poly I:C.

11. The nanoparticle according to any one of claims 7-11, **characterized in that** a molar ratio of the compound of formula I, the nucleic acid adjuvant and the antigen peptide is (50~100): (0~1): (20-50).

12. A preparation method of the nanoparticle according to any one of claims 7 to 12, **characterized by** comprising: a nano-vaccine preparation method comprising: mixing the compound of formula I, nucleic acid adjuvant and the polypeptide antigen with a TM buffer, stirring at 37 °C for 15 min, and then dialyzing in deionized water, to prepare a solution comprising nanoparticles.

13. A nano-vaccine comprising the nanoparticle of any one of claims 6-11 and an adjuvant acceptable in the nano-vaccine.

14. An application of the nano-vaccine of claim 13 in preparation of medicine for prevention and/or treatment of tumor, bacterial infection and/or virus infection.
